# EUROPÄISCHE PATENTSCHRIFT

(11) **EP 1 230 202 B1**
(45) Veröffentlichungstag und Bekanntmachung des Hinweises auf die Patenterteilung: **28.12.2005**
(21) Anmeldenummer: 00927125.5
(22) Anmeldetag: 03.05.2000
(51) Int. Cl.: C07C 17/12, C07C 17/383, C07C 25/18

(54) **VERFAHREN ZUR HERSTELLUNG VON 4-CHLORBIPHENYL**
METHOD FOR PRODUCING 4-CHLOROBIPHENYL
PROCEDE DE PREPARATION DE 4-CHLOROBIPHENYLE

(30) Priorität: 14.05.1999 DE 19922402
(43) Veröffentlichungstag der Anmeldung: 14.08.2002
(73) Patentinhaber: LANXESS Deutschland GmbH, 51369 Leverkusen (DE)
(72) Erfinder: LANGER, Reinhard, D-47918 Tönisvorst (DE); KLAUSENER, Alexander, D-50259 Pulheim (DE); WAGNER, Paul, D-40597 Düsseldorf (DE); PUPPE, Lothar, D-51399 Burscheid (DE)
(86) Internationale Anmeldenummer: PCT/EP2000/003945
(87) Internationale Veröffentlichungsnummer: WO 2000/069798

(56) Entgegenhaltungen:
- FR-A- 2 067 384
- US-A- 1 835 754
- US-A- 1 890 427
- BOTTA, ARTUR ET AL: "Selective p-chlorination of biphenyl in L-zeolites" ANGEW. CHEM. (1991), 103(12), 1687-9 (SEE ALSO ANGEW. CHEM., INT. ED. ENGL., 1991, 30(12), 1689-90) , XP000213809 in der Anmeldung erwähnt

## Beschreibung

Die vorliegende Erfindung betrifft ein Verfahren zur Herstellung von 4-Chlorbiphenyl durch Chlorierung von Biphenyl, und Isolierung des 4-Chlorbiphenyls aus dem Reaktionsgemisch in hoher Reinheit mit niedrigem Gehalt an 3-Chlorbiphenyl.

4-Chlorbiphenyl ist ein wichtiges Zwischenprodukt zur Herstellung von Pharmazeutika und Pflanzenschutz-Produkten. Es ist deshalb notwendig, daß es auf einfache Weise, kostengünstig und in hohen Reinheiten zur Verfügung gestellt werden kann.

Es sind Verfahren zur Herstellung von 4-Chlorbiphenyl bekannt, die relativ komplexe Vorstufen erfordern wie Phenylboronsäure, 4-Chlor-phenyl-diazonium-Salze, Phenylmagnesiumbromid, 4-Brom-chlorbenzol, 4-Iod-chlorbenzol, Phenyltriflat, 4-Brom-biphenyl und Diphenyl-tetrazol-Verbindungen (siehe Org. Chem., 62(10), 3405-6 (1997); Nippon Kagaku Kaishi, 1997 (2), 119-26; Tetrahedron, 52(21), 7201-20 (1996); Org. Chem. 57(1), 391-3 (1992) und J. Chem. Res., Synop. 1994 (6), 216-7). Wenn auch die Ausbeuten teilweise über 90 % liegen und 4-Chlorbiphenyl gut in reiner Form und frei von 3-Chlorbiphenyl isoliert werden kann, so sind doch die Vorstufen nur in sehr aufwendigen Verfahren herstellbar. Deshalb sind diese Verfahren zur Herstellung von 4-Chlorbiphenyl unwirtschaftlich und für eine Durchführung im technischen Maßstab wenig geeignet.

In Tetrahedron 52(26), 8863-6 (1996) wird die Chlorierung von Biphenyl mit 2 Äquivalenten Zinntetrachlorid und 1 Äquivalent Bleitetraacetat beschrieben. Die Ausbeute an 4-Chlorbiphenyl beträgt 70 %. Über die Menge des gebildeten 2- und 3-Chlorbiphenyls und deren Abtrennungsrate wird nichts gesagt. Der Einsatz großer Mengen Bleitetraacetats und Zinntetrachlorids macht das Verfahren aufwendig und unwirtschaftlich.

In Angew. Chem., 103(12), 1687-9 (1991) wird die Chlorierung von Biphenyl, katalysiert durch Zeolithe vom Strukturtyp LTL beschrieben, wobei die Kaliumform (K-L) als besonders günstig bezeichnet wird. Diese Veröffentlichung betrifft jedoch nicht die Herstellung und Isolierung von 4-Chlorbiphenyl, sondern die Herstellung und Isolierung von 4,4'-Dichlorbiphenyl, das aus dem Produktgemisch auskristallisiert werden kann.

Wünschenswert wäre es, 4-Chlorbiphenyl in einem Schritt aus Biphenyl und Chlor herstellen zu können. Dies ist gemäß der US 1 890 427 auch möglich mittels Chlorierung von Biphenyl in Chlorbenzol und in Gegenwart von metallischem Eisen als Katalysator. Dabei fällt jedoch eine Mischung an, die 4-Chlorbiphenyl, 3-Chlorbiphenyl, 2-Chlorbiphenyl und unumgesetztes Biphenyl enthält. Die Trennung dieses Gemisches ist sehr aufwendig. Die Literaturstelle beschreibt für die Abtrennung eines Isomeren die Kristallisation aus nicht-eutektischen Mischungen oberhalb des Gefrierpunktes des Eutektikums. Dies bedeutet, daß man zur Trennung des gesamten Gemisches eine komplizierte Sequenz aus Destillations- und Kristallisationsschritten durchführen muß. Czech. Chem. Prum., 30(10), 529-32 (1980) bestätigt dies. Dort werden, um Reinheiten von größer als 99 % zu erreichen, für die Trennung von 2-, 3- und 4-Chlorbiphenyl enthaltenden Gemischen folgende nacheinander durchzuführende Schritte beschrieben: 1. Destillation, 2. Kristallisation der Fraktionen der Destillation und 3. Rekristallisation der Fraktionen aus der Kristallisation aus Ethanol.

Die Probleme bei der Isolierung von 4-Chlorbiphenyl aus Reaktionsgemischen, wie sie bei der Chlorierung von Biphenyl anfallen, erklären, warum man zur Herstellung von reinem 4-Chlorbiphenyl die eingangs geschilderten komplizierten Synthesewege, in Betracht gezogen hat.

Es besteht deshalb immer noch ein Bedürfnis nach einem Verfahren, das in einfacher Weise erlaubt, aus preiswerten Ausgangsverbindungen 4-Chlorbiphenyl in hoher Reinheit zu gewinnen.

Es wurde nun ein Verfahren zur Herstellung von 4-Chlorbiphenyl gefunden, das dadurch gekennzeichnet ist, daß man Biphenyl und Chlor in Gegenwart von Kernchlorierungs-Katalysatoren umsetzt und das dabei anfallende Reaktionsgemisch einer fraktionierten Destillation unterwirft.

Erfindungsgemäße Kernchlorierungs-Katalysatoren sind Zeolithe des Strukturtyps K-L, bei denen die beweglichen Ionen zu 80 bis 100 %, bevorzugt zu 90 bis 100 %, aus Kaliumionen bestehen.

Der Kernchlorierungs-Katalysator kann z.B. in Mengen zwischen 0,2 und 20 Gew.%, bevorzugt in Mengen zwischen 2 und 12 Gew.%, bezogen auf eingesetztes Biphenyl, eingesetzt werden.

Die erfindungsgemäße Umsetzung kann z.B. bei Temperaturen zwischen 0 und 120°C, bevorzugt bei Temperaturen zwischen 20 und 100°C, durchgeführt werden. Der Druck während der erfindungsgemäßen Umsetzung ist nicht kritisch und kann z.B. zwischen 0,2 und 20 bar liegen. Bevorzugt liegt er zwischen 0,8 und 8 bar. Besonders bevorzugt wird bei Normaldruck chloriert.

Die erfindungsgemäße Umsetzung kann batchweise, semibatchweise oder kontinuierlich durchgeführt werden. Als Batch-Reaktoren sind z.B. Rührkessel, Blasensäulen und Loop-Reaktoren geeignet. Soll das Verfahren kontinuierlich betrieben werden, so kann dies erreicht werden, indem mehrere der obengenannten Batch-Reaktoren in Reihe geschaltet werden. Im Prinzip können aber auch Verweilzeitrohrreaktoren oder Reaktoren zum Betrieb stationärer Katalysatorbetten eingesetzt werden.

Gegebenenfalls kann die erfindungsgemäße Umsetzung in Gegenwart eines Lösungsmittels und/oder eines Cokatalysators durchgeführt werden. Geeignete Lösungsmittel sind z.B. aprotische Lösungsmittel, die unter den Reaktionsbedingungen nicht mit Chlor reagieren. Bevorzugt sind chlorierte Kohlenwasserstoffe, insbesondere Methylenchlorid. Als Co-Katalysator kommt z.B. Chloressigsäure in Frage. Die erfindungsgemäße Chlorierung wird im allgemeinen so durchgeführt, daß vom eingesetzten Biphenyl 30 bis 90 %, bevorzugt 10 bis 70 %, besonders bevorzugt 30 bis 60 %, im geraden Durchgang umgesetzt werden.

Beim erfindungsgemäßen Verfahren wird das nach der Chlorierung vorliegende Reaktionsgemisch durch eine fraktionierte Destillation in die Komponenten aufgetrennt.

Der heterogene Kernchlorierungs-Katalysator wird vor der Destillation zweckmäßigerweise mechanisch abgetrennt, z.B. durch Filtration oder Zentrifugieren.

Vor der Destillation kann dem zu destillierenden Gemisch eine Base zugesetzt werden, um eventuell vorhandene Reste von katalytischer Aktivität zu zerstören und damit zuverlässig Transchlorierungen während der Destillation zu verhindern. Als Basen für diesen Zweck kommen z.B. Natriumcarbonat, Calciumcarbonat, Natriumacetat und Kaliumhydrogencarbonat in Frage.

Die Kolonnen zur Fraktionierung des gegebenenfalls wie oben beschrieben behandelten Reaktionsgemisches aus der Chlorierung können z.B. zwischen 5 und 500, bevorzugt zwischen 10 und 100, besonders bevorzugt zwischen 20 und 50 theoretische Böden aufweisen. Wie diese theoretische Bodenzahl durch Kolonneneinbauten erreicht werden kann, ist dem Fachmann bekannt. Die Kolonnen werden beispielsweise mit einem Rücklaufverhältnis zwischen 1 und 30, bevorzugt zwischen 2 und 20, betrieben.

Man kann die Kolonnen z.B. mit Sumpftemperaturen von 100 bis 300°C, vorzugsweise 150 bis 250°C, und bei Drucken beginnend im Bereich 0,5 bis 3 bar und endend bei 3 bis 300 mbar, vorzugsweise 10 bis 100 mbar, betreiben.

Die fraktionierte Destillation kann batchweise, semibatchweise oder kontinuierlich durchgeführt werden.

Ein kontinuierliches Verfahren zur fraktionierten Destillation kann z.B. so aussehen, daß drei hintereinander geschaltete Kolonnen, die mit Abtriebs- und Verstärkerstufen ausgerüstet sind, betrieben werden. Die erste Kolonne liefert als Kopfprodukt unumgesetztes Biphenyl, die zweite 2-Chlorbiphenyl und die dritte 4-Chlorbiphenyl.

Eine diskontinuierliche fraktionierte Destillation kann man mit einer Kolonne der oben beschriebenen Art durchführen, die nicht mit einer Abtriebs- und einer Verstärkerstufe ausgerüstet ist.

Wenn ein Lösungsmittel verwendet wurde, z.B. Methylenchlorid, so wird dieses zweckmäßigerweise vor der eigentlichen fraktionierten Destillation abgetrennt, z.B. durch Destillation über eine vorgeschaltete Kolonne.

Die erfindungsgemäße Chlorierung führt im allgemeinen zu Reaktionsgemischen, die wenig 3-Chlorbiphenyl enthalten. Das Verhältnis von 4-Chlorbiphenyl zu 3-Chlorbiphenyl im Reaktionsgemisch liegt beispielsweise bei über 100:1, häufig über 300:1 und besonders günstig bei über 800:1. Sollte es ausnahmsweise bei 100:1 oder darunter liegen, sollte man durch routinemäßige Reihenversuche eine Kombination von Reaktionsbedingungen, Katalysator und gegebenenfalls Lösungsmittel ermitteln, bei der die gewünschten geringen Gehalte an 3-Chlorbiphenyl im Reaktionsgemisch erhalten werden können. Mit der erfindungsgemäß durchzuführenden fraktionierten Destillation gelingt es immerhin, den Gehalt von 3-Chlorbiphenyl im isolierten 4-Chlorbiphenyl, wenn auch nicht unter die Nachweisgrenze, so doch merklich zu senken.

Andererseits kann man mit dem erfindungsgemäßen Verfahren auf einfache Weise, aus einfachen Ausgangsmaterialien und mit einfachen Hilfsstoffen 4-Chlorbiphenyl erhalten, das Reinheiten von 99,5 % und mehr, häufig von 99,95 % und mehr, aufweist. Es ist damit gut für die Weiterverarbeitung zu Pharmazeutika und Pflanzenschutz-Produkten geeignet. Die Ausbeuten an 4-Chlorbiphenyl liegen beim erfindungsgemäßen Verfahren bei über 90 %.

Mit dem erfindungsgemäßen Verfahren wird erstmals 4-Chlorbiphenyl zur Verfügung gestellt, das zwischen 0,001 und 10 ‰, vorzugsweise zwischen 0,01 und 1 ‰ und besonders bevorzugt zwischen 0,03 und 0,3 ‰, 3-Chlorbiphenyl enthält. Es ist ein typisches Merkmal des erfindungsgemäßen Verfahrens, daß es 4-Chlorbiphenyl mit diesen Gehalten an 3-Chlorbiphenyl liefert.

### Beispiele

Allgemeines
a) Zur quantitativen Bestimmung der Komponenten wurden 5 %ige Lösungen der Reaktionsmischungen in Chlorbenzol gaschromatographisch analysiert.
   Die Nachweisgrenzen waren: 200 ppm Biphenyl, 100 ppm Monochlorbiphenyl, 400 ppm 4,4'-Dichlorbiphenyl und 400 ppm Trichlorbiphenyle.
b) Reaktor: Ölthermostatisierter 250 ml Planschlifftopf mit Gaseinleitrohr und Rührer.

### Beispiel 1

### Chlorierung in Methylenchlorid

93,5 g Biphenyl wurden in 134 g Methylenchlorid vorgelegt und 9 g Zeolith des Typs K-L (trocken) als feines Pulver zugegeben. Der Reaktor wurde mit Stickstoff gespült und bei 40°C mit der Chloreinleitung begonnen. Einzelheiten sind aus Tabelle 1 ersichtlich.

**Tabelle 1**

| Chloreinleitungszeit (min) | Chlormenge | Gehalt an Reaktionsgemisch (F1.-%) | | | | | Selektivität 4-Chlorbiphenyl (%) |
|---|---|---|---|---|---|---|---|
| | | Biphenyl | Chlorbiphenyl | | | | |
| | | | 2- | 3- | 4- | 4,4'-Di | |
| 45 | 28,7 | 76,92 | 0,74 | 0,01 | 21,59 | 0,58 | 93,54 |
| 80 | 47,2 | 61,33 | 1,21 | 0,04 | 35,29 | 1,83 | 91,26 |
| 108 | 62,8 | 48,94 | 1,58 | 0,04 | 45,46 | 3,60 | 90,34 |
| 125 | 70,5 | 43,22 | 1,76 | 0,06 | 49,79 | 4,74 | 87,69 |

### Beispiel 2

### Chlorierung in Gegenwart von Chloressigsäure

101 g Biphenyl, 2,5 g Chloressigsäure und 10 g Zeolith des Typs K-L (trocken) wurden vorgelegt und mit Stickstoff gespült. Dann wurde auf 100°C erwärmt und mit der Einleitung von Chlor begonnen. Einzelheiten sind aus Tabelle 2 ersichtlich.

**Tabelle 2**

| Chloreinleitungszeit (min) | Chlormenge | Gehalt an Reaktionsgemisch (F1.-%) | | | | | Selektivität 4-Chlorbiphenyl (%) |
|---|---|---|---|---|---|---|---|
| | | Biphenyl | Chlorbiphenyl | | | | |
| | | | 2- | 3- | 4- | 4,4'-Di | |
| 240 | 31,8 | 79,55 | 2,51 | 0,08 | 16,07 | 0,31 | 78,58 |
| 425 | 58,7 | 59,75 | 5,41 | 0,12 | 30,03 | 1,27 | 74,61 |

### Beispiel 3

Destillation (alle Prozentangaben sind Gewichtsprozente, soweit nichts anderes angegeben ist. CBP steht für Chlorbiphenyl).

Entsprechend Beispiel 1 wurde eine größere Menge Reaktionsgemisch der Zusammensetzung hergestellt, wie sie im Beispiel 1 nach 108 Minuten Chlorierzeit vorlag. Aus dem Reaktionsgemisch wurde zunächst der Katalysator durch Abfiltrieren und der größte Teil des Methylenchlorids durch Abziehen über eine Kolonne abgetrennt. Es lagen dann 2991,5 g eines Gemisches vor, das
7,45 % Methylenchlorid,
45,86 % Biphenyl,
1,62 % 2-CBP,
0,12 % 3-CBP,
41,5 % 4-CBP und
3,44 % 4,4'-DICBP enthielt.

Dieses Gemisch wurde über eine 34 theoretische Böden aufweisende Kolonne (Innendurchmesser 5 cm, Höhe 100 cm, Füllkörper 4x4 mm Maschendrahtringe) aus einem 4 I-Sumpfkolben fraktioniert destilliert.

Zunächst wurden bei Normaldruck und bis auf 500 mbar erniedrigtem Druck, bei Sumpftemperaturen von 202 bis 210°C, Kopftemperaturen von 39 bis 24°C und einem Rücklaufverhältnis von 5 56,8 g Methylenchlorid erhalten.

Dann wurden bei einem Druck am Kopf der Kolonne von 30 mbar, bei Sumpftemperaturen von 156 bis 178°C, Kopftemperaturen von 136 bis 137°C und einem Rücklaufverhältnis von 5 1209,4 g Biphenyl erhalten. Dieses kann zur Chlorierung zurückgeführt werden.

Nun wurden bei einem Druck der Kolonne am Kopf von 30 mbar, bei Sumpftemperaturen von 178 bis 179°C, Kopftemperaturen von 138 bis 166°C und einem von 5 auf 10 steigenden Rücklaufverhältnis 254,9 g einer Zwischenfraktion erhalten, die Biphenyl, 2-CBP, 3-CBP und 4-CBP enthielt. Diese kann in die Destillation zurückgeführt werden.

Jetzt wurden bei einem Druck der Kolonne am Kopf von 30 mbar, einer Sumpftemperatur von 180°C, einer Kopftemperatur von 166°C und einem Rücklaufverhältnis von 10 351,6 g einer ersten 4-CBP-Fraktion erhalten, die 99,7 % 4-CBP, 0,16 % 3-CBP und jeweils unter 0,1 % 2-CBP und Biphenyl enthielt. Diese Fraktion kann, wenn die Spezifikation ausreichend ist, als Produkt, gegebenenfalls im Gemisch mit der zweiten 4-CBP-Fraktion, abgegeben werden, oder, wenn die Spezifikation nicht ausreichend ist, in die Destillation zurückgeführt werden.

Schließlich wurden bei einem Druck der Kolonne am Kopf von 30 mbar, Sumpftemperaturen von 181 bis 231°C, einer Kopftemperatur von 166°C und einem Rücklaufverhältnis von 2 675,5 g einer zweiten 4-CBP-Fraktion erhalten, die über 99,95 % 4-CBP und 0,03 % 3-CBP enthielt.

## Patentansprüche

1. Verfahren zur Herstellung von 4-Chlorbiphenyl, **dadurch gekennzeichnet, daß** man Biphenyl und Chlor in Gegenwart eines Zeolithen des Typs L als Kernchlorierungs-Katalysator umsetzt, bei dem die beweglichen Ionen zu 80 bis 100 % aus Kaliumionen bestehen und das dabei anfallende Reaktionsgemisch einer fraktionierten Destillation unterwirft.

2. Verfahren nach Anspruch 1, **dadurch gekennzeichnet, daß** der Kernchlorierungs-Katalysator in einer Menge von 0,2 bis 20 Gew.%, bezogen auf eingesetztes Biphenyl, eingesetzt wird.

3. Verfahren nach Anspruch 1 oder 2, **dadurch gekennzeichnet, daß** man die Umsetzung bei Temperaturen zwischen 0 und 120°C und Drucken im Bereich 0,2 bis 20 bar durchführt.

4. Verfahren nach Ansprüchen 1, 2 oder 3, **dadurch gekennzeichnet, daß** man für die fraktionierte Destillation Kolonnen mit 5 bis 500 theoretischen Böden einsetzt und diese bei einem Rücklaufverhältnis zwischen 1 bis 30 betreibt.

5. Verfahren nach Ansprüchen 1, 2, 3 oder 4, **dadurch gekennzeichnet, daß** man unabhängig voneinander die Umsetzung und die fraktionierte Destillation batchweise, semibatchweise oder kontinuierlich durchführt.

## Claims

1. A process for preparing 4-chlorobiphenyl, **characterized in that** biphenyl and chlorine are reacted in the presence of a zeolite of the type as ring-chlorination catalyst in which the mobile ions are from 80 to 100% potassium ions and the reaction mixture obtained is subjected to fractional distillation.

2. A process as claimed in claim 1, **characterized in that** the ring-chlorination catalyst is used in an amount of from 0.2 to 20% by weight, based on biphenyl used.

3. A process as claimed in claims 1 or 2, **characterized in that** the reaction is carried out at temperatures of from 0 to 120°C and pressures in the range from 0.2 to 20 bar.

4. A process as claimed in claims 1, 2 or 3 **characterized in that** the fractional distillation is carried out using columns having from 5 to 500 theoretical plates and operated at a reflux ratio in the range from 1 to 30.

5. A process as claimed in claims 1, 2, 3 or 4, **characterized in that** the reaction and the fractional distillation are, independently of one another, carried out batchwise, semibatchwise or continuously.

## Revendications

1. Procédé pour la préparation du 4-chlorobiphényle, **caractérisé en ce que** l'on fait réagir le biphényle et le chlore en présence d'une zéolite du type L qui sert de catalyseur de chloruration dans les noyaux et pour laquelle les ions mobiles consistent pour 80 à 100 % en ions potassium, après quoi on soumet le mélange de réaction à une distillation fractionnée.

2. Procédé selon revendication 1, **caractérisé en ce que** le catalyseur de chloruration dans les noyaux est mis en oeuvre en quantité de 0,2 à 20 % du poids du biphényle mis en oeuvre.

3. Procédé selon la revendication 1 ou 2, **caractérisé en ce que** la réaction est réalisée à des températures de 0 à 120°C sous des pressions dans l'intervalle de 0,2 à 20 bar.

4. Procédé selon les revendications 1, 2 ou 3, **caractérisé en ce que**, pour la distillation fractionnée, on utilise des colonnes à 5 à 500 plateaux théoriques et on les fait opérer à un rapport de reflux de 1 à 30.

5. Procédé selon les revendications 1, 2, 3 ou 4, **caractérisé en ce que** la réaction et la distillation fractionnées sont réalisées, indépendamment l'une de l'autre, en discontinu, en semi-continu ou en continu.
